# EUROPEAN PATENT SPECIFICATION

(11) **EP 2 957 614 B1**
(45) Date of publication and mention of the grant of the patent: **29.11.2017**
(21) Application number: 15760362.2
(22) Date of filing: 25.03.2015
(51) Int. Cl.: C09K 3/16, C08K 5/20, C08K 5/55, C08J 5/18, C07C 233/36, C07F 5/04

(54) **ANTISTATIC AGENT, MOLDED ARTICLE COMPRISING INSULATOR POLYMER MATERIAL, AND METHOD FOR PRODUCING SAME**
ANTISTATISCHES MITTEL, FORMKÖRPER MIT ISOLATIONSPOLYMERMATERIAL UND VERFAHREN ZUR HERSTELLUNG DAVON
AGENT ANTISTATIQUE, ARTICLE MOULÉ COMPRENANT UN MATÉRIAU POLYMÈRE ISOLANT ET PROCÉDÉ POUR LE PRODUIRE

(30) Priority: 10.04.2014 JP 2014080883
(43) Date of publication of application: 23.12.2015
(73) Proprietor: Boron Laboratory Co., Ltd., Yachiyo-shi, Chiba 276-0028 (JP)
(72) Inventor: HAMANAKA, Hiroyoshi, Yachiyo-shi Chiba 276-0027 (JP)
(74) Representative: Schwabe - Sandmair - Marx
(86) International application number: PCT/JP2015/059133
(87) International publication number: WO 2015/156133

(56) References cited:
- EP-A2- 0 337 664
- CN-A- 101 029 151
- JP-A- H04 183 716
- JP-A- H04 252 287
- JP-A- H04 288 390
- JP-A- H04 311 735
- JP-A- S61 238 839
- JP-A- 2011 079 918
- JP-A- 2014 082 487
- JP-A- 2014 083 750

## Description

### Technical Field

The present invention relates to an antistatic agent for insulating polymer materials, e.g., plastics and synthetic fibers, to a molded article composed of an insulating polymer material that uses this antistatic agent, and to a method of producing this molded article.

### Background Art

The antistatic agents used for insulating polymer products, e.g., plastic molded articles and synthetic fiber products, include surface coating-type antistatic agents, which treat the product surface. With a surface coating-type antistatic agent, any of various surfactants is dissolved as such in a volatile solvent or is dissolved or dispersed in a volatile solvent in combination with an easily adhesive polymer compound vehicle. The application of this surface coating-type antistatic agent to the surface of a target product causes a strengthening of the polarity of the outermost region of the target product and thereby reduces the generation of static electricity.

However, when a surfactant-based antistatic agent is coated by itself on the surface of an insulating polymer product, the antistatic performance immediately after the treatment is excellent, but, due to a weak surface adsorption force, perturbations in the polarity are produced in a short period of time and the performance then deteriorates. In addition, when a surfactant-based antistatic agent is applied in combination with an easily adhesive polymer compound vehicle, the composition of the mixture makes it difficult to efficiently raise the polarity of the outermost region. Furthermore, while the intended antistatic performance is not obtained unless a fair amount of the antistatic agent is admixed, the adhesiveness is weakened when the mixing proportion for the antistatic agent is increased and as a consequence the electrical properties at the surface end up becoming unstable.

Research has also been carried out through the ages into internally kneaded-type antistatic agents, which, through preliminary admixture of surfactant into the starting material for the plastic molding or synthetic fiber product, are targeted to the long-lasting antistatic performance (refer, for example, to Nonpatent Document 1). However, the matrix constituting the polymer product target is actually not a single solid and has crystalline regions and amorphous regions. Due to this, the surfactant that is assumed to exert the antistatic performance by migrating from the interior to the surface is not fixed to the surface, and a weaker antistatic performance is then exhibited than for the surface coating-type antistatic agents and in addition the durability of the antistatic effect is also not as would be expected.

In the case of polymer blend-type antistatic agents, a selection that exhibits compatibility is made from polymer compounds that have an ionic or nonionic polar group inserted in main chain or side chain position, and this is melt-mixed with the starting material for the insulating polymer product followed by molding. The goal here has been to obtain - through crosslinking of this polymer blend-type antistatic agent with the main chain of the polymer starting material - a stable and long-lasting antistatic performance beyond that of surfactant-based antistatic agents. In this case, however, an antistatic effect is not obtained at small amounts of incorporation due to the major influence of the amount of polar groups distributed in the outermost region of the insulating polymer material, and an amount that is 5- to 20-times that of the aforementioned internally kneaded-type antistatic agent is then necessary. As a consequence, the cost of producing the polymer products is driven up and changes in the properties of the polymer starting material must also be taken into account in any potential use.

On the other hand, there is strong demand, centered in the electric and electronic industries in particular, for static electricity countermeasures that can securely and reliably prevent the malfunction and failure of IC devices without using a conductive material, e.g., a metal or electrically conductive carbon, and that at the same time are economical, convenient, and highly reproducible. The previously described surfactant-based antistatic agents and polymer blend-type antistatic agents cannot respond to this demand due to the risk of ionic impurities coming closer to the main body of the IC device during the expression of their antistatic effect.

The present inventor therefore invented an internally kneaded-type donor-acceptor hybrid-type antistatic agent that unlike the conventional antistatic agents described above, just by incorporation in about the same amount as a surfactant-based antistatic agent, produces a hole transport action within the matrix that constitutes the insulating polymer product; that unlike a polymer blend-type antistatic agent does not alter the properties of the polymer material target; and that provides a highly reproducible antistatic effect without being accompanied by a large cost increase. This internally kneaded-type donor-acceptor hybrid-type antistatic agent has been the subject of a patent application (Patent Document 1).

However, while this antistatic agent is superior to the prior antistatic agents, it is still not satisfactory with respect to the requirements imposed by IC devices. Thus, as described below, due to differences in the structure of the antistatic agent and the state of dispersion based thereon, the remarkable antistatic effect as in the present invention is not obtained.

An invention related to an antistatic polyolefin resin composition is disclosed in Patent Document 2; however, the antistatic agent here, as seen from its description, is a neutralization product between an amine and an ionic bond-type organoboron compound and is not a donor-acceptor type molecular compound and there is thus of course also no teaching relative to a donor-acceptor type molecular compound that generates the remarkable effect of the present invention

### Prior Art Documents

### Patent Documents

Patent Document 1: Japanese Patent Publication No. 2011-079918 A
Patent Document 2: Japanese Patent Publication No. S 61-238839 A

### Nonpatent Documents

Nonpatent Document 1: Hiroyoshi HAMANAKA, "Antistatic agents for synthetic resins", Journal of Japan Cosmetic Chemists Association, Vol. 7, No. 1, p. 28 (1971).

### Summary of Invention

### Problems that the Invention is to Solve

The internally kneaded-type donor-acceptor hybrid-type antistatic agent according to Patent Document 1 is superior to the prior antistatic agents, but is unable to reliably protect IC devices in which malfunction or failure is produced by a very small amount of static electricity. In particular, molded articles composed of an insulating polymer material readily generate static electricity. Accordingly, an object of the present invention is to provide an antistatic agent composed of a donor-acceptor type molecular compound that can prevent static charge on an insulating polymer material surely with good persistency and reproducibility. Further objects of the present invention are to provide a molded article composed of an insulating polymer material that uses this antistatic agent and to provide a method of producing such a molded article.

### Means for Solving the Problems

These problems are solved by the following inventions 1) to 3).
1) An antistatic agent for insulating polymer materials, characterized in that the antistatic agent is composed of a donor-acceptor type molecular compound represented by general formula (1) below and obtained by **mixing, melting,** and reacting a semi-polar organoboron compound (donor component) of the upper part in the general formula (1) below with a basic nitrogen compound (acceptor component) of the lower part in the general formula (1) below. 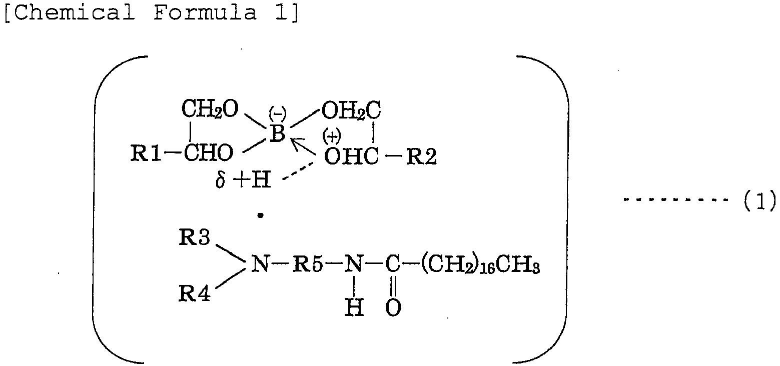
   In the above formula, R1 and R2 are each independently CH₃(CH₂)₁₆-CO-OCH₂ or HOCH₂ and at least one thereof is CH₃(CH₂) ₁₆-CO-OCH₂; R3 and R4 are each independently CH₃, C₂H₅, HOCH₂, HOC₂H₄, or HOCH₂CH(CH₃); and R5 is C₂H₄ or C₃H₆.
2) A method of producing a molded article comprising an insulating polymer material, characterized in that the method comprises: melt-dispersing the antistatic agent according to 1) in an insulating polymer material that has been heated to equal to or higher than its glass transition temperature; and carrying out molding in a molding apparatus having a steel surface wherein the insulating polymer material is brought into contact with the steel surface of the molded apparatus and at the surface of the molded article, an antistatic film in which the antistatic agent is arrayed is formed.
3) A molded article comprising an insulating polymer material, characterized in that the molded article is produced by the production method according to 2) and has, at its surface, the antistatic film in which the antistatic agent composed of the donor-acceptor type molecular compound represented by the above general formula (1) is arrayed.

### Advantageous Effects of Invention

The present invention can provide a donor-acceptor type antistatic agent that can prevent static charge on an insulating polymer material surely with good persistency and reproducibility. The present invention can also provide a molded article composed of an insulating polymer material that uses this antistatic agent and can further provide a method of producing such a molded article.

Moreover, this antistatic agent - because it has the ability to continuously and efficiently attenuate the static electricity produced at a molded article composed of an insulating polymer material at both the surface and in the interior of the molded article - may be mixed in just small amounts into the insulating polymer material used for molding and is thus very convenient and economical.

### Brief Description of Drawings

FIG. 1 is a diagram that shows the IR absorption spectrum of the semi-polar organoboron compound donor component obtained in Example 1;
FIG. 2 is a diagram that shows the IR absorption spectrum of the donor-acceptor type molecular compound obtained in Example 1; and
FIG. 3 is a diagram that shows the IR absorption spectrum of the internally kneaded-type donor-acceptor hybrid-type antistatic agent of Comparative Example 19.

### Mode for Carrying Out the Invention

The present invention is particularly described hereinbelow.

As a result of intensive investigations, the present inventor discovered that a superior antistatic performance with respect to insulating polymer materials is possessed by a donor-acceptor type molecular compound provided by the reaction of the semi-polar organoboron compound moiety of the upper part in general formula (1) as the donor component and the tertiary amine moiety of the lower part in general formula (1) as the acceptor component at a molar ratio between the two of approximately 1 : 1. The present invention was achieved based on this discovery.

With regard to this donor component, the "δ+" indicates that polarity is present in the covalent bond within a molecule; the (+) indicates the strong electron-donating character of the oxygen atom; the (-) indicates the strong electron-withdrawing character of the boron atom; the "→" indicates the path of electron attraction; and the "---" indicates a state in which the bonding force between atoms is weakened.

The donor component here must be limited to glycerol residues that have one or two C₁₇ straight-chain saturated hydrocarbon groups so as to bring the area occupied by the semi-polar-bonded atomic group to a minimum in the crystalline state.

The acceptor component here must be made a tertiary amine in which one of the N-substituents is a group having a C₁₇ straight-chain saturated hydrocarbon group bonded at the end thereof through an amide bond and in which the two remaining N-substituents are C₁₋₃ hydrocarbon groups or hydroxyhydrocarbon groups.

When the donor component or acceptor component does not satisfy these structural requirements, the effects of the present invention cannot be obtained - even by similar or analogous structures.

In addition, prior to mixture with the insulating polymer material, the donor-acceptor type molecular compound with the general formula (1) must be prepared in advance by **mixing, melting,** and reacting the donor component and the acceptor component at a molar ratio of approximately 1 : 1. When the donor component and acceptor component are simply separately mixed into the insulating polymer material without being reacted in advance, the probability that the two components will react within the mixed system is very small, and as a consequence the formation of the above-mentioned molecular compound will be almost nonexistent and the effects of the present invention can then not be obtained. Moreover, the mixing ratio between the two components is preferably as near to 1 : 1 as possible. A satisfactory formation of the molecular compound occurs when the mixing ratio between the two components is as near to 1 : 1 as possible, and the effects of the present invention are then thoroughly expressed. The mixing ratio is preferably in the range from 1 : 0.8 to 0.8 to 1.

The antistatic agent of the present invention is used by being melt-dispersed in an insulating polymer material that has been heated to at least the glass transition temperature. The molding apparatus used to mold the insulating polymer material is preferably a molding apparatus that has a steel surface that comes into contact with the polymer material during the molding process. Molding causes the antistatic agent to become even smaller crystalline particles and stably disperse within the molded article and thereby increases the hole transport action sites. Antistatic agents that use the prior surfactants remain as micelles, even with molding, and are not monodispersed, and such an effect is then not obtained. Furthermore, in the case of the antistatic agent of the present invention, the heated and melted insulating polymer material undergoes almost no heat-induced changes of the material and in addition comes into contact with a steel surface, which has an excellent dimensional stability. Due to this, antistatic agent molecules present at the interface between the insulating polymer material and the steel surface collectively form a strong solid adsorption film due to Van der Waals forces and intermolecular hydrogen bonding forces, thus forming, at the surface of the molded article, an antistatic film in which the antistatic agent is arrayed. As a result, the generation and retention of static electricity do not occur, even under severe conditions, and a molded article is obtained that can also be used for IC devices.

Example 17 in Patent Document 1 uses a donor-acceptor hybrid-type antistatic agent with a composition composed of the same components as for the present invention. However, in this Example 17, the donor component and the acceptor component are added separately to methyl methacrylate monomer and polymerization is carried out after melt-mixing. Due to this, because the donor component and acceptor component are simply separately incorporated in the resin in the state of a composition, there is almost no formation of the donor-acceptor type molecular compound as given by the general formula (1) of the present invention. Furthermore, because monomer casting molding is carried out in the above Example 17, the insulating polymer material does not come into contact with a steel surface and an antistatic film as in the present invention is not formed on the surface of the molded article. Moreover, the remarkable antistatic effects as in the present invention are also not obtained in the other examples because - in addition to the almost complete absence of formation as in Example 17 of the donor-acceptor type molecular compound - these are donor component/acceptor component combinations different from that in the present invention.

The antistatic agent of the present invention has the following characteristic features based on its chemical structure.
- Because a glycerol triester is used as the structural skeleton for the donor component, crystallization can occur in a state in which the area occupied by the molecule is small.
- The crystallinity is strengthened still further due to the combination of the acceptor component and donor component that have the same straight-chain saturated hydrocarbon group of optimal chain length.
- A tough and stable antistatic film can be produced due to the formation within the crystal structure of intermolecular hydrogen bonds by the amide linkage groups.

Because the antistatic agent of the present invention has these characteristic features, it can also cause charge leakage to occur at the molded article surface in addition to the hole transport action in the interior of the molded article. As a result, and as shown in the examples provided below, an antistatic effect is obtained that is remarkably better than that in the prior art.

The semi-polar organoboron compound moiety that is the donor component can be synthesized by the following methods.

A 2 : 1 complete ester (triester) is prepared by reacting 1 mole of boric acid with 2 moles of glycerol, or the 2 : 1 complete ester (triester) is produced by reacting 1 mole of a lower alcohol-boric acid triester with 2 moles of glycerol. The semi-polar organoboron compound moiety is then obtained by carrying out an esterification reaction or a transesterification reaction, using straight-chain stearic acid or a straight-chain stearic acid ester of a lower alcohol, on the 1 or 2 alcoholic OH groups remaining in the product.

The reaction temperature during these serial reactions is suitably 50 to 250°C at normal pressure, and the use of a catalyst is not particularly required.

In accordance with another method, the 2 : 1 complete borate ester (the triester) may be synthesized by reacting 1 mole of boric acid or a lower alcohol-boric acid triester with 2 moles of a glyceryl monostearate as an intermediate raw material that has been prepared in advance. The reaction temperature in this case is suitably 100 to 200°C at normal pressure, and the use of a catalyst is not particularly required.

The tertiary amine moiety that is the acceptor component can be synthesized by the following method (1) or (2).
(1) Amidation is performed by reacting 1 mole of a straight-chain stearic acid or a straight-chain stearic acid ester of a lower alcohol with 1 mole of a diamine in which a primary amino group is linked by a C₂ or C₃ alkylene group with a tertiary amino group that is substituted by hydrocarbon groups having 1 to 3 carbons.
(2) 2 moles of formaldehyde, ethylene oxide, or propylene oxide is reacted with 1 mole of a diamine in which a primary amino group and a straight-chain stearyl amide group are linked by a C₂ or C₃ alkylene group.

The temperature of the amidation reaction is suitably 100 to 230°C at normal pressure, and the use of a catalyst is not required.

The temperature of the tertiarization reaction for generating the tertiary amino group is suitably 100 to 150°C at normal pressure, and the use of a catalyst is not required.

The aforementioned solid semi-polar organoboron compound and the aforementioned solid tertiary amine are then stirred and mixed while heating; heating and stirring are stopped when they have dissolved in each other; and the antistatic agent composed of the donor-acceptor type molecular compound of the present invention is then obtained by cooling and solidification.

This donor-acceptor type molecular compound according to the present invention is a molecular compound of nonionic partner substances and is different from the salt of the ionic bond-type organoboron compound of Patent Document 2. This can be confirmed by the fact that, when the molecular compound is made into the THF solution and subjected to gel permeation analysis carried by passage through a polystyrene gel, the molecular weight is not increased and peaks for the molecular weight peaks of the semi-polar organoboron compound and the tertiary amine appear in a form in which they are combined as such.

### Examples

The present invention is more specifically described below through the examples and comparative examples provided below, but the present invention is not limited to or by these examples. Unless specifically indicated otherwise, "parts" and "%" in the examples denote "mass parts" and "mass%".

### < Example 1 >

2 Moles of glycerol and 1 mole of boric acid were placed in a four-neck flask that was positioned in a heating device and was provided with a stirrer, thermometer, nitrogen gas inflow tube, and a condenser-equipped water measurement tube, and dehydration of 3 moles of water was completed by stirring and heating to 210°C at normal pressure under a nitrogen gas inflow. 2 Moles of straight-chain stearic acid was subsequently introduced and a fatty acid esterification reaction was then carried out at 100 to 250°C and normal pressure under a nitrogen gas inflow and dehydration of additional 2 moles of water was completed, thereby finishing the reaction.

After the production of the semi-polar organoboron compound donor component had been confirmed based on the appearance of the absorption for the bending vibration in the IR absorption spectrum analysis (refer to (2) below) of the OH group that forms the semi-polar bond, which is observed prominently at 830 to 835 cm⁻¹, cooling was carried out to lower the temperature of the contents to 70°C.

The relationship between the appearance of this bending vibration absorption and the production of the semi-polar organoboron compound has been described in "Studies on Semipolar Organoboron Surfactants", Hiroyoshi HAMANAKA, Journal of Japan Oil Chemists' Society (Yukagaku), Vol.29, No. 12, pages 893-895 (1980).

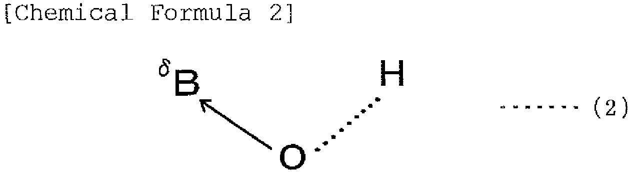

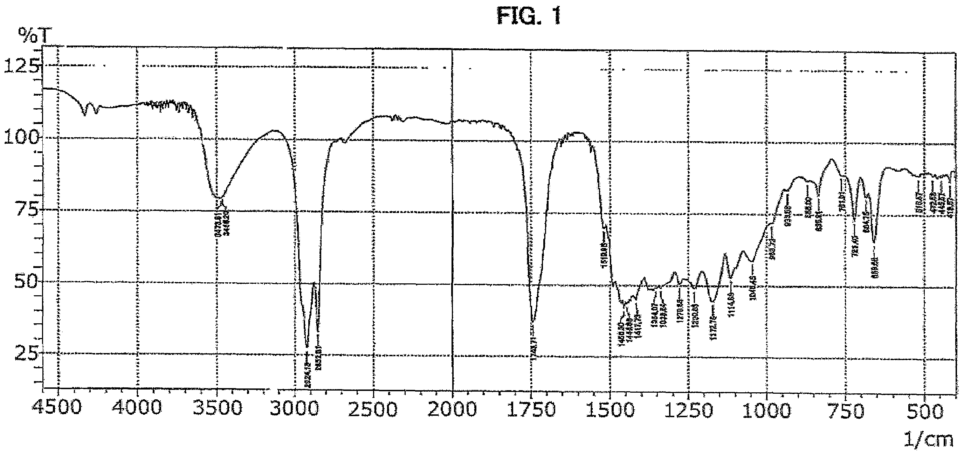

The IR absorption spectrum of the aforementioned semi-polar organoboron compound measured by the KBr pellet method is shown in FIG. 1, and a peak is shown to be present in the vicinity of 835 cm⁻¹.

In addition, the acid value of due to the structural dislocation (hereinafter, "structural dislocation acid value") of this semi-polar organoboron compound measured by potentiometric titration analysis was 77.6 (theoretical value = 77.4).

Separately, 1 mole of dimethylaminopropylamine and 1 mole of straight-chain stearic acid were introduced into a four-neck flask set up as described above and an amidation reaction was carried out at 100 to 230°C under normal pressure and a nitrogen gas inflow; dehydration of water in an amount corresponding to 1 mole was completed to obtain a tertiary amine acceptor component.

The amine value of this tertiary amine measured by neutralization titrimetric analysis was 152.8 (theoretical value = 152.2).

1 mole of the tertiary amine was then added to 1 mole of the aforementioned semi-polar organoboron compound and the two components were dissolved to uniformity in each other and reacted at 70 to 80°C and normal pressure followed by casting onto a flat stainless steel plate cooled to 20°C to obtain the antistatic agent with the following formula (3).

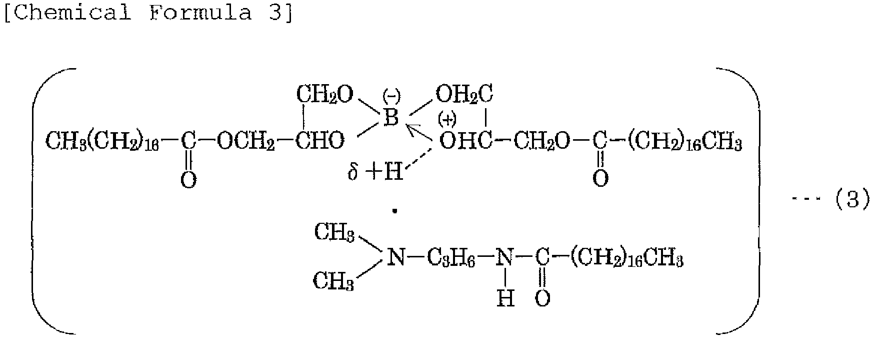

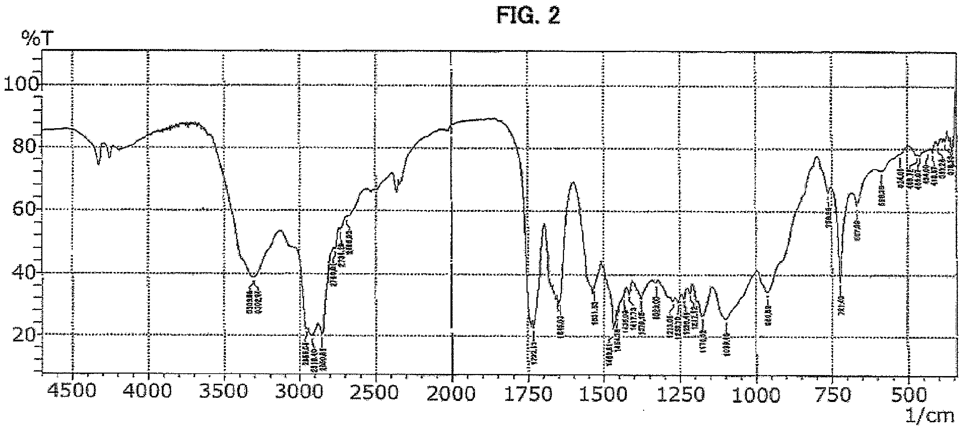

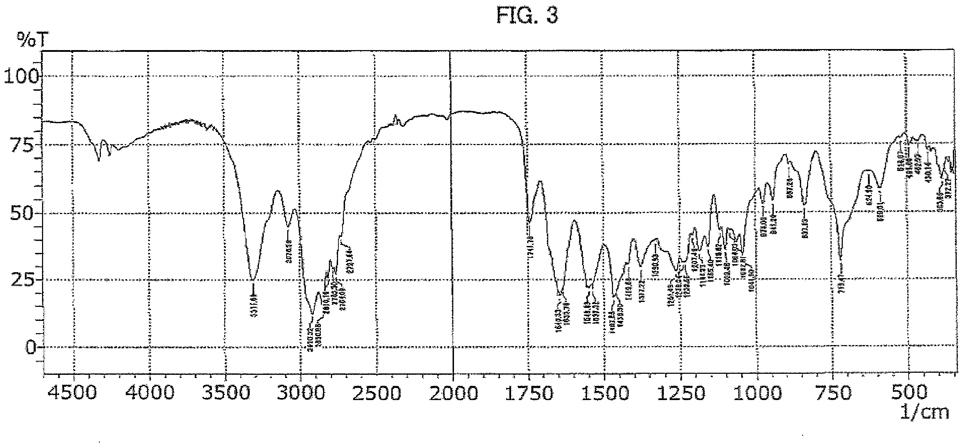

The IR absorption spectrum of the obtained antistatic agent measured by the KBr pellet method is shown in FIG. 2, and it could be confirmed based on the following facts [1] to [3] that a donor-acceptor type molecular compound according to the present invention had been obtained.
[1] The peak in the vicinity of 835 cm⁻¹ for the semi-polar organoboron compound has been extinguished.
   Extinction of the peak is not seen in the IR absorption spectrum of a composition provided by simply mixing the semi-polar organoboron compound and the basic nitrogen compound (refer to FIG. 3), as in the Comparative Example 19 given below (this corresponds to the internally kneaded-type donor-acceptor hybrid-type antistatic agent of Patent Document 1).
[2] The absorption at 1732 cm⁻¹ for the stretching vibration of the "C=O" of the ester bond and the absorption band at 1645 cm⁻¹ for the stretching vibration of the "C=O" in the amide bond can be observed, which shows that both the semi-polar organoboron compound moiety and the tertiary amine moiety are present.
[3] Because the absorption for the stretching vibration of the OH group of the semi-polar organoboron compound moiety observed in the vicinity of 3500 cm⁻¹ in FIG. 1 is wholly shifted to longer wavelengths to around 3300 cm⁻¹ in FIG. 2, it can be confirmed that intermolecular forces are acting between the semi-polar organoboron compound moiety and the tertiary amine moiety.

The presence of the donor-acceptor type molecular compounds in the examples and comparative examples that follow was similarly confirmed by IR absorption spectrum analysis.

### < Example 2 >

2 Moles of glycerol and 1 mole of boric acid were introduced into a four-neck flask set up as in Example 1 and were stirred and heated to 210°C and dehydration of 3 moles of water was completed. 1 Mole of methyl straight-chain stearate was then introduced; a transesterification reaction was carried out at 230 to 240°C; and methyl alcohol in an amount corresponding to 1 mole was distilled from the system to obtain a semi-polar organoboron compound donor component. The production of this boron compound was confirmed by IR absorption spectrum analysis in the same manner as described in Example 1. In addition, the structural dislocation acid value of this boron compound, measured in the same manner as in Example 1, was 122.2 (theoretical value = 122.3).

Separately, an amidation reaction was carried out between 1 mole of diethylaminopropylamine and 1 mole of straight-chain stearic acid using a four-neck flask set up as described above, and dehydration of water in an amount corresponding to 1 mole was completed to obtain a tertiary amine acceptor component. The amine value of this tertiary amine, measured in the same manner as in Example 1, was 141.5 (theoretical value = 141.4).

1 mole of this tertiary amine was then added to 1 mole of the aforementioned semi-polar organoboron compound and the two components were dissolved in each other and reacted at 80 to 90°C followed by casting onto a flat stainless steel plate cooled to 20°C to obtain an antistatic agent with the following formula (4).

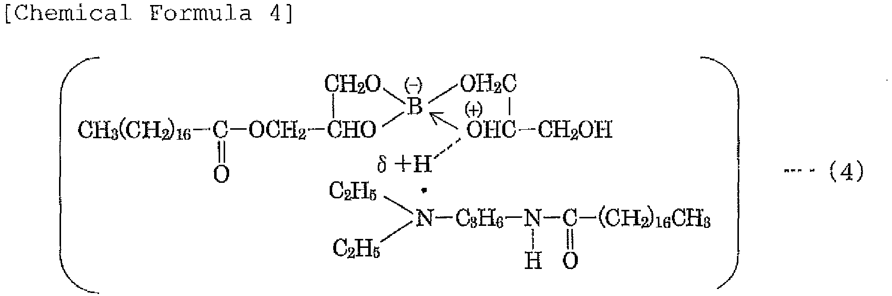

### < Example 3 >

2 Moles of glyceryl monostearate and 1 mole of triethyl borate were introduced into a four-neck flask set up as in Example 1 and a transesterification reaction was carried out at 100 to 200°C and ethyl alcohol in an amount corresponding to 3 moles was distilled out of the system to obtain a semi-polar organoboron compound donor component. The production of this boron compound was confirmed by IR absorption spectrum analysis as in Example 1. In addition, the structural dislocation acid value of this boron compound, measured in the same manner as in Example 1, was 77.5 (theoretical value = 77.4).

Separately, a tertiary amine acceptor component was obtained by reacting 2 moles of ethylene oxide in the absence of a catalyst with 1 mol of the monoamide of stearic acid with ethylenediamine. The amine value of this tertiary amine, measured in the same manner as in Example 1, was 135.2 (theoretical value = 135.3).

1 mole of this tertiary amine was then added to 1 mole of the aforementioned semi-polar organoboron compound and the two components were dissolved to uniformity in each other and reacted at 80 to 90°C followed by casting onto a flat stainless plate cooled to 20°C to obtain an antistatic agent of the present invention with the following formula (5).

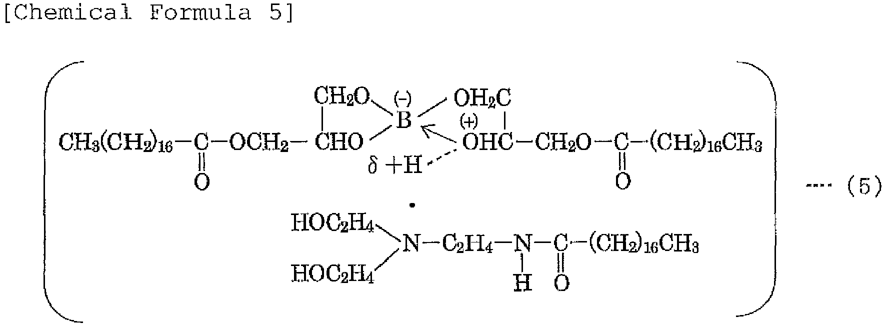

### < Examples 4 to 8 >

Antistatic agents were obtained in the same manner as in Example 1, but changing the materials for synthesis of the semi-polar organoboron compound or the tertiary amine such that R1 to R5 in the general formula (1) provided the structures shown in the following formulas (6) to (10). Formulas (6) to (10) are the antistatic agents of Examples 4 to 8, respectively.

The structural dislocation acid value of the semi-polar organoboron compound constituent and the amine value of the tertiary amine constituent of each antistatic agent were measured in the same manner as in Example 1 and are shown below each of the formulas. 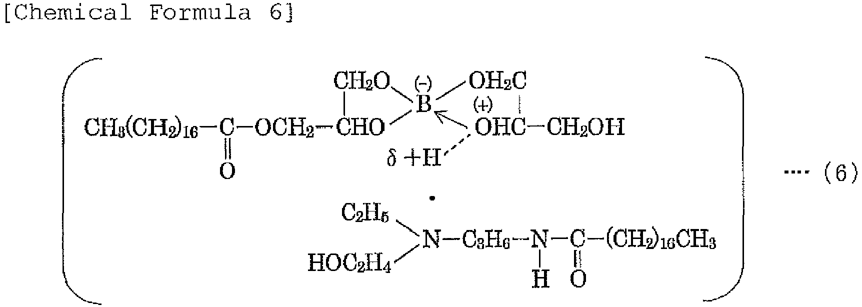
- structural dislocation acid value of the semi-polar organoboron compound: 122.2 (theoretical value = 122.3)
- amine value of the tertiary amine: 136.2 (theoretical value = 136.0)

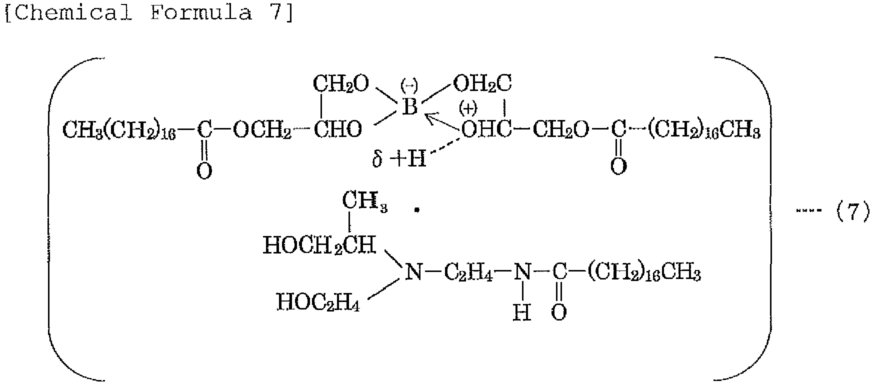

- structural dislocation acid value of the semi-polar organoboron compound: 77.5 (theoretical value = 77.4)
- amine value of the tertiary amine: 130.8 (theoretical value = 130.9)

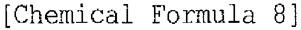

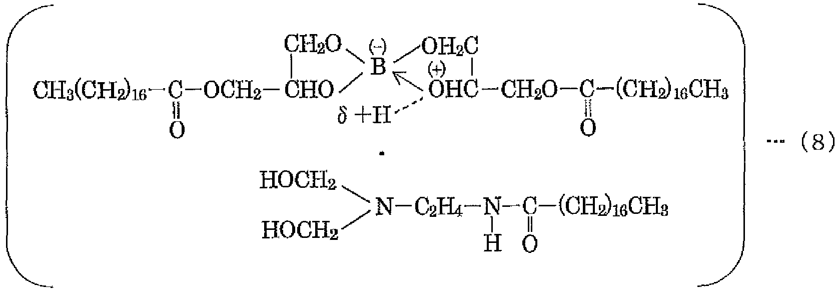

- structural dislocation acid value of the semi-polar organoboron compound: 77.5 (theoretical value = 77.4)
- amine value of the tertiary amine: 145.3 (theoretical value = 145.1)

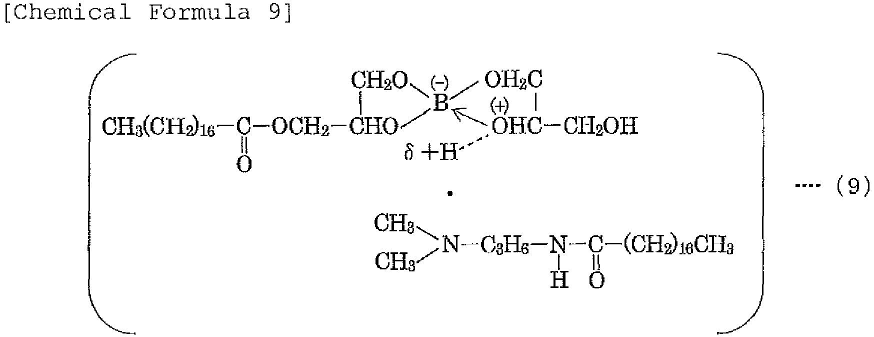

- structural dislocation acid value of the semi-polar organoboron compound: 122.2 (theoretical value = 122.3)
- amine value of the tertiary amine: 152.2 (theoretical value = 152.2)

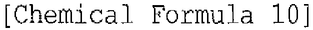

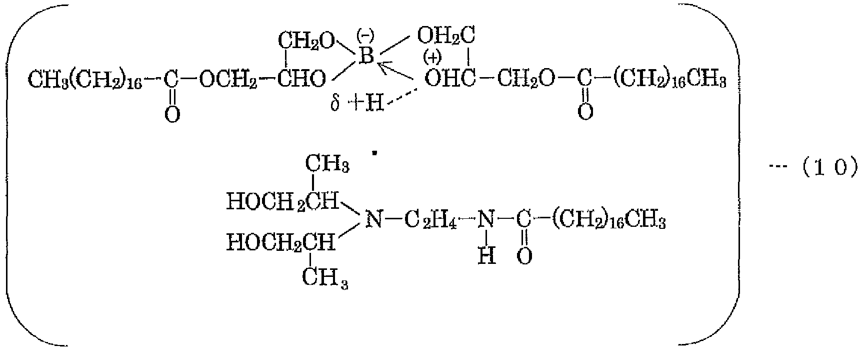

- structural dislocation acid value of the semi-polar organoboron compound: 77.5 (theoretical value = 77.4)
- amine value of the tertiary amine: 126.7 (theoretical value = 126.8)

Antistatic agents of Comparative Examples 1 to 8 were obtained in the same manner as in Example 1, but changing the material for synthesis of the semi-polar organoboron compound and/or the tertiary amine such that the moieties corresponding to the donor component or acceptor component in the general formula (1) had the structures shown in formulas (11) to (18) below.

The structural dislocation acid value of the semi-polar organoboron compound constituent and the amine value of the tertiary amine constituent of each antistatic agent were measured in the same manner as in Example 1 and are shown below each formula.

### < Comparative Example 1 >

This is an example of the use of a carbon chain for the R1 and R2 straight-chain saturated hydrocarbon groups that are shorter than in the semi-polar organoboron compounds of the examples. 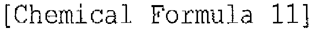 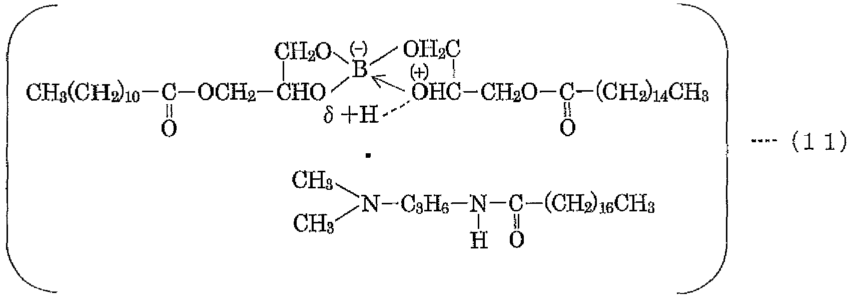
- structural dislocation acid value of the semi-polar organoboron compound: 91.7 (theoretical value = 91.6)
- amine value of the tertiary amine: 152.2 (theoretical value = 152.2)

### < Comparative Example 2 >

This is an example of the use of a carbon chain for the R1 and R2 straight-chain saturated hydrocarbon groups that are longer than in the semi-polar organoboron compounds of the examples. 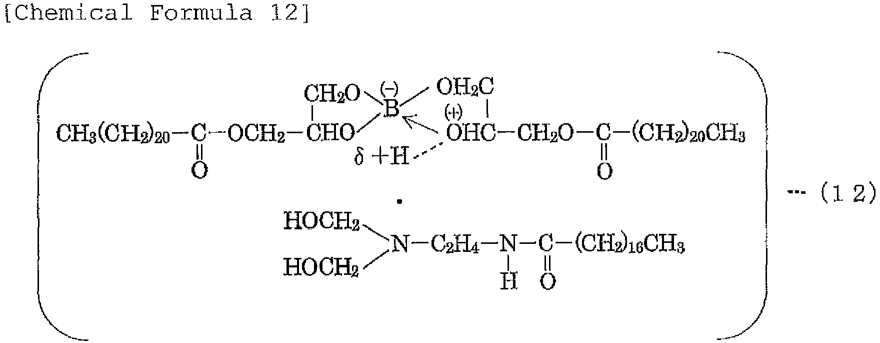
- structural dislocation acid value of the semi-polar organoboron compound: 67.1 (theoretical value = 67.0)
- amine value of the tertiary amine: 145.3 (theoretical value = 145.1)

### < Comparative Example 3 >

This is an example in which the polyhydric alcohol residues in the semi-polar organoboron compound are larger than glycerols and the area occupied by the molecule is therefore larger. 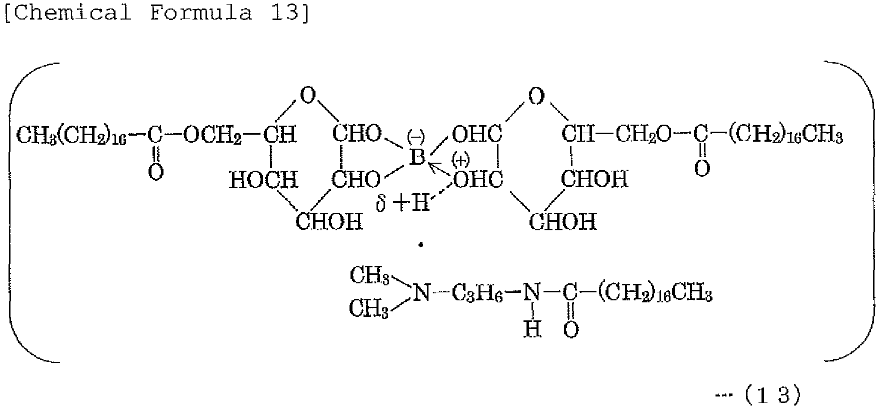
- structural dislocation acid value of the semi-polar organoboron compound: 62.2 (theoretical value = 62.3)
- amine value of the tertiary amine: 152.2 (theoretical value = 152.2)

### < Comparative Example 4 >

This is an example in which the tertiary amine does not have an amide bond. 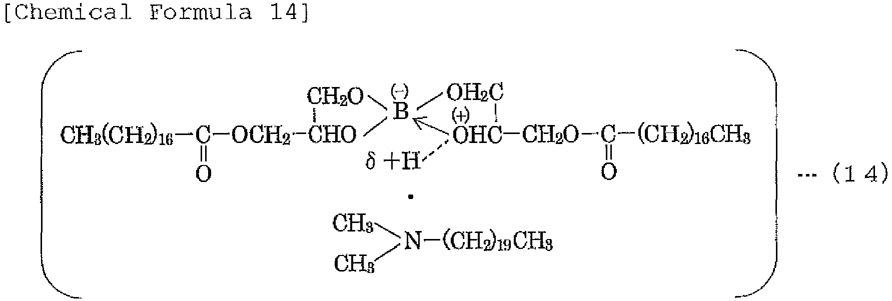
- structural dislocation acid value of the semi-polar organoboron compound: 77.5 (theoretical value = 77.4)
- amine value of the tertiary amine: 172.2 (theoretical value = 172.3)

### < Comparative Example 5 >

This is an example that has a short carbon chain for the straight-chain saturated hydrocarbon group at the end connected to the amide bond. 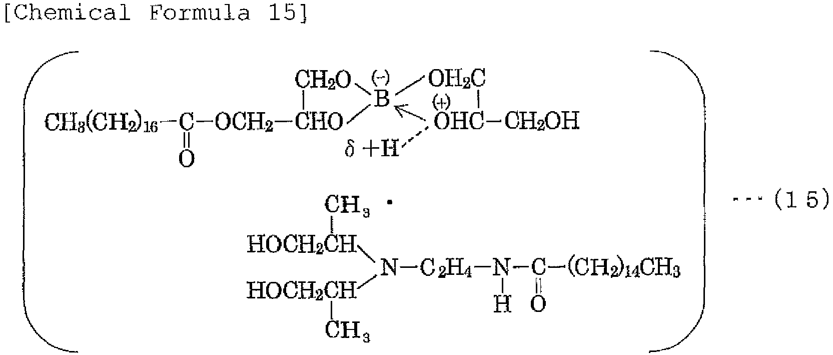
- structural dislocation acid value of the semi-polar organoboron compound: 122.2 (theoretical value = 122.3)
- amine value of the tertiary amine: 135.5 (theoretical value = 135.3)

### < Comparative Example 6 >

This is an example that has a long carbon chain for the straight-chain saturated hydrocarbon group at the end connected to the amide bond. 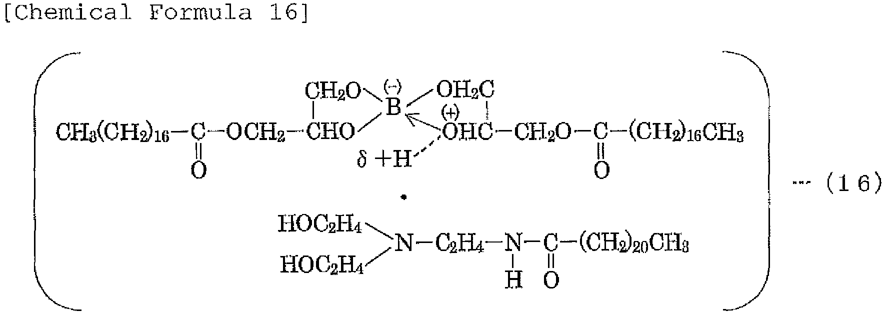
- structural dislocation acid value of the semi-polar organoboron compound: 77.5 (theoretical value = 77.4)
- amine value of the tertiary amine: 119.4 (theoretical value = 119.2)

### < Comparative Example 7 >

This is an example in which the tertiary amine has two hydrocarbon groups in which a C₁₇ saturated hydrocarbon group is bonded at the end across an amide bond. 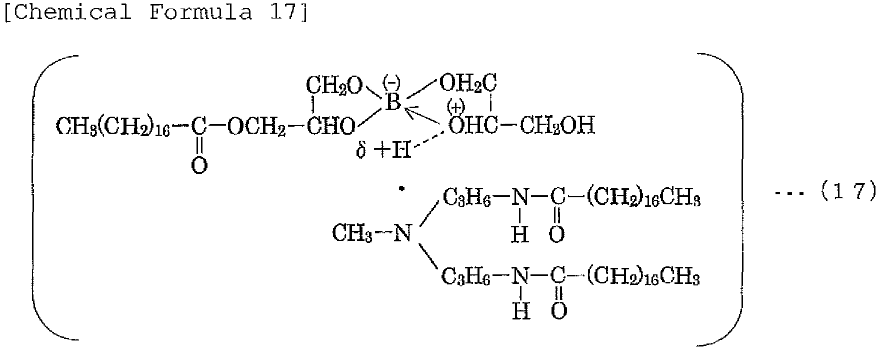
- structural dislocation acid value of the semi-polar organoboron compound: 122.2 (theoretical value = 122.3)
- amine value of the tertiary amine: 82.8 (theoretical value = 82.7)

### < Comparative Example 8 >

This is an example in which the acceptor component is a secondary amine. 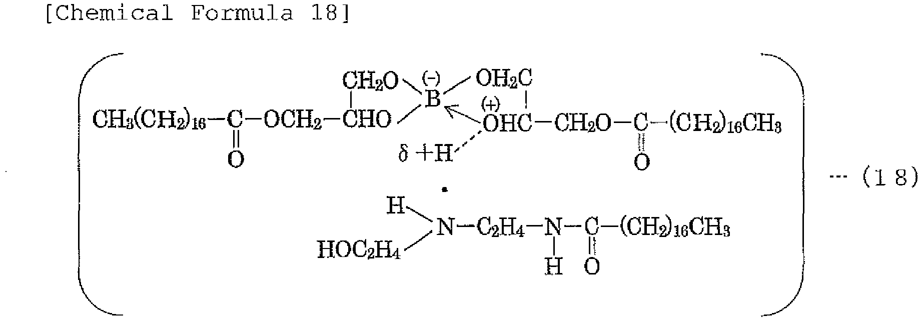
- structural dislocation acid value of the semi-polar organoboron compound: 77.5 (theoretical value = 77.4)
- amine value of the secondary amine: 151.5 (theoretical value = 151.4)

### < Examples 11 to 18 and Comparative Examples 11 to 19 >

Using the antistatic agents of Examples 1 to 8 and Comparative Examples 1 to 8, transparent polyethylene films of Examples 11 to 18 and Comparative Examples 11 to 18 were respectively fabricated as described in the following.

More specifically, masterbatches each containing 10% of each antistatic agent in a low-density polyethylene (Suntec LD-F 22254 from Asahi Kasei Chemicals Corporation) were prepared, and 2 parts of each masterbatch and 98 parts of the same low-density polyethylene were uniformly mixed followed by inflation molding at 165°C to produce a 20 µm-thick transparent polyethylene film that contained 0.2% of the antistatic agent.

This 0.2% content is an amount lower than the ordinary amount of the internally kneaded-type antistatic agents added for polyethylenes.

Comparative Example 19 is an example in which the internally kneaded-type donor-acceptor hybrid-type antistatic agent used in Example 17 of Patent Document 1 (this is a composition that contains the same donor component and acceptor component as in the present invention, but each in a free state) was melt-mixed into the same low-density polyethylene as in Example 11.

Each of the transparent polyethylene films of Examples 11 to 18 and Comparative Examples 11 to 19 was held for 6 months under conditions of 15°C and 40% RH, after which the specific surface resistance of each transparent polyethylene film was measured under the same temperature and humidity conditions. In addition, forced charging was performed by the application of a voltage of 5000 V, and the charge attenuation rate was measured after 2 seconds after the application was halted. The measurements were performed using a Hiresta UP-MCP-HT450 surface resistance analyzer from Mitsubishi Chemical Analytech Co., Ltd. and a Static Honestmeter from Shishido Electrostatic Ltd.

The results are given in Table 1: it is seen that, with reference to the examples, the specific surface resistance is remarkably lower than in the comparative examples and charging is completely absent. The specific surface resistance of the low-density polyethylene film in the absence of antistatic agent addition is at least
10¹⁵ Ω/□.

**[Table 1]**

| | | Antistatic agent Formula No. | Specific surface resistance (Ω/□) | Charge attenuation rate (%) |
|---|---|---|---|---|
| Examples | 11 | (3) | 4.0 × 10⁹ | 100 |
| | 12 | (4) | 5.1 × 10⁹ | 100 |
| | 13 | (5) | 5.2 × 10⁹ | 100 |
| | 14 | (6) | 4.3 × 10⁹ | 100 |
| | 15 | (7) | 5.0 × 10⁹ | 100 |
| | 16 | (8) | 8.1 × 10⁹ | 100 |
| | 17 | (9) | 6.3 × 10⁹ | 100 |
| | 18 | (10) | 5.0 × 10⁹ | 100 |
| Comparative Examples | 11 | (11) | 4.2 × 10¹² | 85 |
| | 12 | (12) | 1.0 × 10¹³ | 80 |
| | 13 | (13) | 3.2 × 10¹³ | 75 |
| | 14 | (14) | 6.3 × 10¹³ | 63 |
| | 15 | (15) | 5.1 × 10¹² | 88 |
| | 16 | (16) | 3.3 × 10¹² | 85 |
| | 17 | (17) | 7.8 × 10¹³ | 57 |
| | 18 | (18) | 3.7 × 10¹⁴ | 39 |
| | 19 | - | 4.1 × 10¹⁴ | 37 |

### < Examples 21 to 28 and Comparative Examples 21 to 28 >

Using the antistatic agents of Examples 1 to 8 and Comparative Examples 1 to 8, transparent polyethylene bottles of Examples 21 to 28 and Comparative Examples 21 to 28 were respectively produced as described in the following.

More specifically, masterbatches were each produced that contained 10% of each antistatic agent in linear low-density polyethylene (Novatec LL-UE320 from Japan Polyethylene Corporation), and 10 parts of each masterbatch and 90 parts of the same linear low-density polyethylene were uniformly mixed. This was followed by blow molding at 165°C to produce transparent polyethylene bottles that had a 1.0% content of the antistatic agent and a thickness of 500 µm, a height of 18 cm, a bottom diameter of 7 cm, and a top end diameter of 2 cm.

### < Comparative Example 29 >

An transparent polyethylene bottle was produced in the same manner as in Example 21, but changing the antistatic agent to the glyceryl monostearate surfactant-based antistatic agent given by the following formula (19).

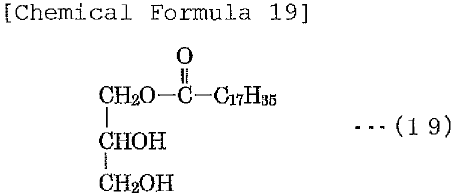

The transparent polyethylene bottles of Examples 21 to 28 and Comparative Examples 21 to 29 were left to stand for 1 year under conditions of 45°C and 50% RH and then removed and the specific surface resistance of the side surface of each bottle was measured under conditions of 23°C and 50% RH using the same instrumentation as in Example 11. In addition, the adhesion state of paper fragments was visually inspected after rubbing 20 times with a cotton cloth under a load of 300 g. The presence or absence of stickiness at the bottle surface was also checked by touching with a finger.

The results are given in Table 2: it is seen that, with reference to the examples, the specific surface resistance is remarkably lower than in the comparative examples and there is also no adhesion of the paper fragments after rubbing. In addition, even when the content of antistatic agent relative to the polyethylene is increased in the examples, the shortcoming of stickiness at the bottle surface is entirely absent and it is shown that the antistatic performance is reliably and securely retained even during long-term standing at high temperatures. The specific surface resistance of the polyethylene bottle not containing an antistatic agent is at least 10¹⁵ Ω/□.

**[Table 2]**

| | | Antistatic agent Formula No. | Specific surface resistance (Ω/□) | Adhesion of paper fragments | Stickiness at the bottle surface |
|---|---|---|---|---|---|
| Examples | 21 | (3) | 1.0 × 10⁹ | absent | absent |
| | 22 | (4) | 1.6 × 10⁹ | absent | absent |
| | 23 | (5) | 1.5 × 10⁹ | absent | absent |
| | 24 | (6) | 1.2 × 10⁹ | absent | absent |
| | 25 | (7) | 2.0 × 10⁹ | absent | absent |
| | 26 | (8) | 2.5 × 10⁹ | absent | absent |
| | 27 | (9) | 2.7 × 10⁹ | absent | absent |
| | 28 | (10) | 1.7 × 10⁹ | absent | absent |
| Comparative Examples | 21 | (11) | 8.9 × 10¹¹ | present | present |
| | 22 | (12) | 5.0 × 10¹² | present | slightly present |
| | 23 | (13) | 6.3 × 10¹² | present | present |
| | 24 | (14) | 8.2 × 10¹² | present | present |
| | 25 | (15) | 1.0 × 10¹² | present | present |
| | 26 | (16) | 9.1 × 10¹¹ | present | present |
| | 27 | (17) | 8.8 × 10¹² | present | slightly present |
| | 28 | (18) | 9.5 × 10¹² | substantial adhesion | present over entire surface |
| | 29 | (19) | 1.3 × 10¹³ | substantial adhesion | present over entire surface |

### < Examples 31 to 38 and Comparative Examples 31 to 38 >

Using the antistatic agents of Examples 1 to 8 and Comparative Examples 1 to 8, transparent polyethylene bottles of Examples 31 to 38 and Comparative Examples 31 to 38 were respectively produced as described below.

More specifically, a high-density polyethylene (Prime Polymer Co., Ltd.: Evolue H) containing 2% of each of the antistatic agent was pulverized and then vacuum-formed at 170°C to produce a cylinder with a thickness of 3 mm, a height of 20 cm, and a true diameter of 10 cm.

### < Comparative Example 39 >

A cylinder was produced for Comparative Example 39 in the same manner as in Example 31, but changing the antistatic agent to the surfactant-based antistatic agent given by formula (19) and used in Comparative Example 29.

### < Comparative Example 40 >

A cylinder was produced for Comparative Example 40 in the same manner as in Example 31, but changing the antistatic agent to the surfactant-based antistatic agent N,N-di(2-hydroxyethyl)octadecylamine given by the following formula (20) .

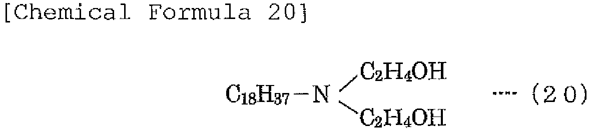

Each of the cylinders of Examples 31 to 38 and Comparative Examples 31 to 40 were placed horizontally and left to stand for 6 months in the natural environment, where the air temperature varied in the range from 0°C to 35°C and the weather was a combination of clear, cloudy, and rain. This was followed by leaving to stand for 48 hours under conditions of 23°C and 50% RH and measurement, using the same instrumentation as in Example 11, of the specific surface resistance of the side surface and bottom of each cylinder under the same temperature and humidity conditions.

The results are given in Table 3: it is seen that, with reference to the examples, the specific surface resistances for both the side surface and the bottom are remarkably lower than in the comparative examples and the properties do not change even when the cylinders of the examples were put for long period of time in the natural environment. As may be understood from Comparative Examples 39 and 40, in the vacuum-forming method, an internally kneaded-type surfactant-based antistatic agent is entirely unable to exhibit an antistatic action, while the donor-acceptor type molecular compound according to the present invention provides excellent effects. The specific surface resistance of the high-density polyethylene cylinder not containing an antistatic agent is at least 10¹⁵ Ω/□.

**[Table 3]**

| | | Antistatic agent Formula No. | Specific surface resistance | |
|---|---|---|---|---|
| | | | Side surface part (Ω/□) | Bottom part (Ω/□) |
| Examples | 31 | (3) | 6.5 × 10⁸ | 5.3 × 10⁸ |
| | 32 | (4) | 7.1 × 10⁸ | 6.8 × 10⁸ |
| | 33 | (5) | 7.2 × 10⁸ | 6.8 × 10⁸ |
| | 34 | (6) | 6.6 × 10⁸ | 5.5 × 10⁸ |
| | 35 | (7) | 7.0 × 10⁸ | 6.8 × 10⁸ |
| | 36 | (8) | 9.1 × 10⁸ | 7.3 × 10⁸ |
| | 37 | (9) | 8.7 × 10⁸ | 7.0 × 10⁸ |
| | 38 | (10) | 6.9 × 10⁸ | 5.9 × 10⁸ |
| Comparative Examples | 31 | (11) | 7.8 × 10¹⁰ | 6.8 × 10¹⁰ |
| | 32 | (12) | 1.3 × 10⁹ | 9.8 × 10¹⁰ |
| | 33 | (13) | 9.7 × 10¹⁰ | 9.0 × 10¹⁰ |
| | 34 | (14) | 2.5 × 10¹¹ | 1.0 × 10¹¹ |
| | 35 | (15) | 9.5 × 10¹⁰ | 9.0 × 10¹⁰ |
| | 36 | (16) | 7.6 × 10¹⁰ | 6.8 × 10¹⁰ |
| | 37 | (17) | 3.2 × 10⁹ | 1.6 × 10⁹ |
| | 38 | (18) | 8.8 × 10¹³ | 8.0 × 10¹³ |
| | 39 | (19) | 8.1 × 10¹³ | 7.8 × 10¹³ |
| | 40 | (20) | 8.8 × 10¹³ | 8.3 × 10¹³ |

### < Examples 41 to 48 and Comparative Examples 41 to 48 >

As described in the following, polypropylene sheets of Examples 41 to 48 and Comparative Examples 41 to 48 were respectively produced using the individual antistatic agents of Examples 1 to 8 and Comparative Examples 1 to 8.

More specifically, masterbatches were each produced that contained 10% of each antistatic agent in polypropylene (random copolymer, Sunallomer PM731H from SunAllomer Ltd.); 10 parts of each masterbatch and 90 parts of the same polypropylene were uniformly mixed; and a 200 µm-thick polypropylene sheet containing 1.0% of the antistatic agent was produced by extrusion molding at 225°C.

### < Comparative Examples 49 and 50 >

The polypropylene sheets of Comparative Examples 49 and 50 were produced in the same manner as in Example 41, but changing the antistatic agent to the surfactant-based antistatic agent given in formula (19) or formula (20).

The polypropylene sheets of Examples 41 to 48 and Comparative Examples 41 to 50 were each left to stand for 1 year under conditions of 10°C and 35% RH, after which the specific surface resistance was measured under the same conditions of temperature and humidity. In addition, forced charging was performed by the application of a voltage of 5000 V and the charge attenuation rate was checked after 2 seconds after the application was halted. The measurements were performed using the same instrumentation as in Example 11.

The results are given in Table 4: it is seen that, with reference to the examples, the specific surface resistance is remarkably lower than in the comparative examples and the charge attenuation is also excellent. Moreover, the antistatic agents of the present invention are seen to reliably keep their antistatic performance even if being left for a long period of time. The specific surface resistance of the polypropylene sheet not containing an antistatic agent addition is at least 10¹⁵ Ω/□.

**[Table 4]**

| | | Antistatic agent Formula No. | Specific surface resistance (Ω/□) | Charge attenuation rate (%) |
|---|---|---|---|---|
| Examples | 41 | (3) | 3.3 × 10⁹ | 100 |
| | 42 | (4) | 4.8 × 10⁹ | 100 |
| | 43 | (5) | 5.0 × 10⁹ | 100 |
| | 44 | (6) | 3.6 × 10⁹ | 100 |
| | 45 | (7) | 5.1 × 10⁹ | 100 |
| | 46 | (8) | 7.8 × 10⁹ | 100 |
| | 47 | (9) | 6.5 × 10⁹ | 100 |
| | 48 | (10) | 4.7 × 10⁹ | 100 |
| Comparative Examples | 41 | (11) | 5.3 × 10¹² | 83 |
| | 42 | (12) | 9.5 × 10¹² | 80 |
| | 43 | (13) | 7.0 × 10¹² | 80 |
| | 44 | (14) | 7.6 × 10¹² | 80 |
| | 45 | (15) | 8.0 × 10¹² | 77 |
| | 46 | (16) | 4.8 × 10¹² | 83 |
| | 47 | (17) | 1.3 × 10¹³ | 65 |
| | 48 | (18) | 7.8 × 10¹³ | 61 |
| | 49 | (19) | 6.0 × 10¹³ | 63 |
| | 50 | (20) | 6.5 × 10¹³ | 63 |

### < Examples 51 to 58 and Comparative Examples 51 to 58 >

As described in the following, polypropylene flat plates of Examples 51 to 58 and Comparative Examples 51 to 58 were respectively produced using the antistatic agents of Examples 1 to 8 and Comparative Examples 1 to 8.

More specifically, 98 Parts of starting material pellets of polypropylene (homopolymer, Sumitomo Noblen AW564 from Sumitomo Chemical Co., Ltd.) and 2 parts of each antistatic agent were uniformly mixed and molded to provide composite pellets, and these were injection molded at 230°C to produce a flat plate having a size of 35 cm × 40 cm × 0.8 cm.

### < Comparative Examples 59 and 60 >

The polypropylene flat plates of Comparative Examples 59 and 60 were produced in the same manner as in Example 51, but changing the antistatic agent to the surfactant-type antistatic agent shown by formula (19) or formula (20).

### < Comparative Example 61 >

10 Parts of the poly(20)oxyethylene glycol/dodecanedioic acid condensation polymer (average degree of polymerization = 15) given by formula (21) below were added as a separately prepared polymer blend-type antistatic agent to 90 parts of the same starting material pellets of polypropylene as in Example 51, and this was injection molded at 230°C to produce the flat plate of Comparative Example 61, which had the same size as in Example 51.

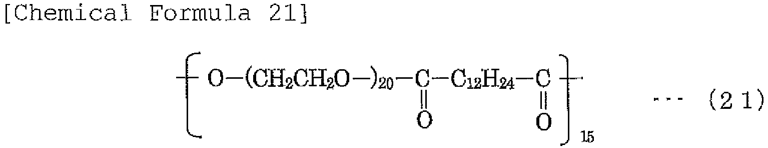

The polypropylene flat plates of Examples 51 to 58 and Comparative Examples 51 to 61 were each left to stand for 1 year at a location where the temperature varied in the range from 0°C to 40°C and the humidity varied in the range from 30 to 70% RH, after which the electrostatic adsorption state of dust was checked. This was followed by transfer to a location where the conditions were 23°C and 50% RH and allowing to stand for 24 hours, and the specific surface resistance of each polypropylene flat plate was then measured using the same instrumentation as in Example 11. In addition, the adhesion state of paper fragments was visually inspected after rubbing 20 times with a cotton cloth under a load of 300 g.

The results are given in Table 5: it is seen that, with reference to the examples, the specific surface resistance is remarkably lower in the examples than in the comparative examples, the electrostatic adsorption of dust is entirely absent, and paper fragment adhesion is also absent. Injection-molded polypropylene articles are used as various industrial and commercial products and household products, but, as may be understood from Comparative Examples 59 to 61, the prior surfactant-type antistatic agents and polymer blend-type antistatic agents do not provide a satisfactory antistatic effect on the long-term. The specific surface resistance of the polypropylene flat plate not containing an antistatic agent addition is at least 10¹⁵ Ω/□.

**[Table 5]**

| | | Antistatic agent Formula No. | Electrostatic adsorption of dust | Specific surface resistance (Ω/□) | Adhesion of paper fragments |
|---|---|---|---|---|---|
| Examples | 51 | (3) | entirely absent | 4.6 × 10⁸ | absent |
| | 52 | (4) | entirely absent | 5.0 × 10⁸ | absent |
| | 53 | (5) | entirely absent | 5.0 × 10⁸ | absent |
| | 54 | (6) | entirely absent | 5.2 × 10⁸ | absent |
| | 55 | (7) | entirely absent | 6.1 × 10⁸ | absent |
| | 56 | (8) | entirely absent | 7.7 × 10⁸ | absent |
| | 57 | (9) | entirely absent | 8.3 × 10⁸ | absent |
| | 58 | (10) | entirely absent | 6.4 × 10⁸ | absent |
| Comparative Examples | 51 | (11) | thinly present over entire surface | 9.3 × 10¹⁰ | adhesion occurred, but drop-off occurred immediately |
| | 52 | (12) | present over entire surface | 1.0 × 10¹¹ | adhesion occurred, but drop-off occurred immediately |
| | 53 | (13) | present over entire surface | 1.3 × 10¹¹ | adhesion occurred, but drop-off occurred immediately |
| | 54 | (14) | present over entire surface | 2.4 × 10¹¹ | adhesion occurred, but drop-off occurred immediately |
| | 55 | (15) | present over entire surface | 1.0 × 10¹¹ | adhesion occurred, but drop-off occurred immediately |
| | 56 | (16) | thinly present over entire surface | 9.8 × 10¹⁰ | adhesion occurred, but drop-off occurred immediately |
| | 57 | (17) | present over entire surface | 3.5 × 10¹¹ | adhesion occurred, but drop-off occurred immediately |
| | 58 | (18) | conspicuous over entire surface | 4.6 × 10¹³ | substantial adhesion |
| | 59 | (19) | conspicuous over entire surface | 3.1 × 10¹³ | substantial adhesion |
| | 60 | (20) | conspicuous over entire surface | 8.6 × 10¹² | substantial adhesion |
| | 61 | (21) | conspicuous over entire surface | 8.0 × 10¹³ | substantial adhesion |

### < Examples 71 to 78 and Comparative Examples 71 to 78 >

As described in the following, ABS resin rectangular boxes of Examples 71 to 78 and Comparative Examples 71 to 78 were respectively produced using the antistatic agents of Examples 1 to 8 and Comparative Examples 1 to 8.

More specifically, 98 Parts of starting material pellets of ABS resin (Toyolac 700 from Toray Industries, Inc.) and 2 parts of each antistatic agent were uniformly mixed and molded to produce composite pellets, and, using these composite pellets, a 1 mm-thick rectangular box with a size of 10 cm × 30 cm × 10 cm was produced by injection molding at 230°C.

### < Comparative Examples 79 to 81 >

The ABS resin rectangular boxes of Comparative Examples 79 to 81 were produced in the same manner as in Example 71, but changing the antistatic agent to the surfactant-type antistatic agent given by formula (19) or formula (20) or the polymer blend-type antistatic agent given by formula (21).

The ABS rectangular boxes of Examples 71 to 78 and Comparative Examples 71 to 81 were each left to stand for 3 months under conditions of 50°C and 75% RH, followed by measurement, using the same instrumentation as in Example 11, of the specific surface resistance of the bottom part under conditions of 23°C and 50% RH. Each ABS resin rectangular box was then held for 1 month in a location where the temperature varied in the range from 10°C to 25°C and the humidity varied in the range from 30 to 60% RH, after which the state of electrostatic adsorption of dust to the side surface was visually inspected.

The results are given in Table 6: it is seen that, with reference to the examples, the specific surface resistance is remarkably lower than in the comparative examples and electrostatic adsorption of dust is entirely absent. In addition, because ABS resin has a complex structure in which main chain composed of saturated hydrocarbon and main chain composed of unsaturated hydrocarbon are intermixed, there is no effect with a prior surfactant-type antistatic agent. Thus, a countermeasure against troubles caused by static electricity has been implemented by the incorporation of polymer blend-type antistatic agents in large amounts. However, an excellent antistatic effect is obtained at small amounts when the antistatic agent of the present invention is used. The specific surface resistance of the ABS rectangular box not containing an antistatic agent is at least 10¹⁵ Ω/□.

**[Table 6]**

| | | Antistatic agent Formula No. | Specific surface resistance (Ω/□) | Ele ctrostatic adsorption of dust |
|---|---|---|---|---|
| Examples | 71 | (3) | 5.1 × 10¹⁰ | entirely absent |
| | 72 | (4) | 6.1 × 10¹⁰ | entirely absent |
| | 73 | (5) | 6.3 × 10¹⁰ | entirely absent |
| | 74 | (6) | 6.1 × 10¹⁰ | entirely absent |
| | 75 | (7) | 7.2 × 10¹⁰ | entirely absent |
| | 76 | (8) | 1.0 × 10¹¹ | entirely absent |
| | 77 | (9) | 1.3 × 10⁹ | entirely absent |
| | 78 | (10) | 7.5 × 10¹⁰ | entirely absent |
| Comparative Examples | 71 | (11) | 4.3 × 10¹³ | present over entire side surface |
| | 72 | (12) | 6.1 × 10¹³ | present over entire side surface |
| | 73 | (13) | 7.0 × 10¹³ | present over entire side surface |
| | 74 | (14) | 7.9 × 10¹³ | present over entire side surface |
| | 75 | (15) | 6.1 × 10¹³ | present over entire side surface |
| | 76 | (16) | 5.5 × 10¹³ | present over entire side surface |
| | 77 | (17) | 5.8 × 10¹³ | present over entire side surface |
| | 78 | (18) | 9.5 × 10¹³ | present over entire side surface |
| | 79 | (19) | 8.7 × 10¹³ | present over entire side surface |
| | 80 | (20) | 9.7 × 10¹² | present over entire side surface |
| | 81 | (21) | 9.5 × 10¹² | present over entire side surface |

### < Examples 91 to 98 and Comparative Examples 91 to 98 >

As described in the following, vinyl chloride resin sheets of Examples 91 to 98 and Comparative Examples 91 to 98 were respectively produced using the antistatic agents of Examples 1 to 8 and Comparative Examples 1 to 8.

More specifically, 0.5 Parts of each antistatic agent was added to a starting material mixture composed of 100 parts of a finely granular vinyl chloride resin (Vinika GF90HA from Mitsubishi Chemical Corporation), 40 parts dioctyl phthalate, 0.5 parts calcium stearate, and 0.5 parts zinc stearate, and a 0.8 mm-thick sheet was produced by calender extrusion molding at 165°C.

### < Comparative Examples 99 and 100 >

The vinyl chloride resin sheets of Comparative Examples 99 and 100 were produced in the same manner as described in Example 91, but changing the antistatic agent to the surfactant-based antistatic agent given by formula (19) or (20).

The vinyl chloride resin sheets of Examples 91 to 98 and Comparative Examples 91 to 100 were each left to stand for 6 months in a space at 70°C where the humidity was varied over the range from 30 to 70% RH, followed by transfer to a location under conditions of 23°C and 50% RH and measurement of the specific surface resistance using the same instrumentation as in Example 11. In addition, the adhesion state of paper fragments was visually inspected after rubbing 20 times with a cotton cloth under a load of 300 g.

The results are given in Table 7: it is seen that, with reference to the examples, the specific surface resistance is remarkably lower than in the comparative examples and paper fragment adhesion is also absent. Moreover, in the case of vinyl chloride resin products that contain a liquid plasticizer component, the molecules of the added antistatic agent end up being incorporated in the plasticizer and it is then difficult to substantially change the electrical properties of the surface. To date, the admixture of large amounts of a plasticizer component having a structure that exhibits an antistatic behavior has been carried out, and this has driven up the cost. However, an excellent antistatic effect is obtained at a small amount when the antistatic agent of the present invention is used. The specific surface resistance of the soft vinyl chloride resin sheet not containing an antistatic agent is at least 10¹⁴ Ω/□.

**[Table 7]**

| | | Antistatic agent Formula No. | Specific surface resistance (Ω/□) | Adhesion of paper fragments |
|---|---|---|---|---|
| Examples | 91 | (3) | 2.5 × 10¹⁰ | absent |
| | 92 | (4) | 3.2 × 10¹⁰ | absent |
| | 93 | (5) | 3.6 × 10¹⁰ | absent |
| | 94 | (6) | 3.0 × 10¹⁰ | absent |
| | 95 | (7) | 3.3 × 10¹⁰ | absent |
| | 96 | (8) | 5.1 × 10¹⁰ | absent |
| | 97 | (9) | 4.8 × 10¹⁰ | absent |
| | 98 | (10) | 3.1 × 10¹⁰ | absent |
| Comparative Examples | 91 | (11) | 9.7 × 10¹¹ | slight adhesion |
| | 92 | (12) | 1.0 × 10¹² | slight adhesion |
| | 93 | (13) | 2.0 × 10¹² | slight adhesion |
| | 94 | (14) | 3.9 × 10¹² | slight adhesion |
| | 95 | (15) | 1.0 × 10¹² | slight adhesion |
| | 96 | (16) | 9.4 × 10⁹ | slight adhesion |
| | 97 | (17) | 4.0 × 10¹² | slight adhesion |
| | 98 | (18) | 8.0 × 10¹² | substantial adhesion |
| | 99 | (19) | 7.1 × 10¹² | substantial adhesion |
| | 100 | (20) | 7.5 × 10¹² | substantial adhesion |

It has been confirmed in the preceding, in tests carried out on a variety of plastic molded articles produced using a steel molding apparatus, that the antistatic agent of the present invention exhibits a superior charge leakage performance.

The antistatic agent of the present invention far exceeds the performance level of pre-existing antistatic agents and can prevent the malfunction and failure of IC devices and can provide the computer industry with a sense of security. The explosions and fires caused by the static electricity unexpectedly produced in common business and household settings can be almost completely prevented.

Moreover, due to the gap in the triboelectric series, it has been difficult to prevent the phenomenon in which contaminants that float due to their light weight, e.g., human and animal hairs, and dust of organic origin, undergo electrostatic adsorption to insulating polymer material products. However, because the addition of the antistatic agent of the present invention during the molding of insulating polymer material products makes it possible to induce charge leakage at the same time that contact occurs, this will be very useful going forward for improving hygiene and sanitation.

In addition, when, for example, a packaging film is fabricated in which the outside is a plastic film that contains the antistatic agent of the present invention and the inside is an insulating film to which antistatic agent has not been added, because charge does not accumulate on the inside the article to be packaged can be smoothly inserted and removed, while in addition packaging can be carried out without direct contact between the product in the interior and the antistatic agent component.

Also in the case of the reverse of the preceding, i.e., a multilayer molded article in which the inside is a plastic film or sheet that contains the antistatic agent of the present invention and the outside is an insulating film or sheet to which antistatic agent has not been added, the product does not accumulate charge at its surfacemost side and because of this, for example, tableware and playground equipment for infants and children can be made even safer and cleaner.

A multilayer molded article can also be produced in which the middle layer is a plastic molded article that contains the antistatic agent of the present invention and in which a molded article lacking antistatic agent is adhered on both sides thereof.

In the case of foam molded articles, prior antistatic agents have not provided an excellent antistatic performance because they end up being adsorbed in large amounts to the bubbles in the interior of the molded article, thus causing the amount distributed at the surface to be small. On the other hand, foaming may be inhibited when the amount of antistatic agent incorporation is increased. It has thus not been possible to date to produce satisfactory antistatic foam molded articles at good reproducibilities. However, the antistatic agent of the present invention occupies a small area as the molecular compound, it crystallizes and is stably present in the three-dimensional interior of the polymer structure surrounding the bubbles and it can cause leakage of charges by a hole transport action. In addition to this, Van der Waals forces and intermolecular hydrogen bond forces operate multi-plexedly at the two-dimensional surface of the polymer structure, thus conferring an inherent characteristic behavior in which an effective ionically-conductive crystalline thin film is formed. As a consequence of the preceding, a foamed polyethylene or a foamed polystyrene that exhibits an effective antistatic performance can be obtained at small amounts of addition.

Moreover, a highly reproducible antistatic performance can be imparted even to rubbery elastic molded articles in which there are open cell channels.

## Claims

1. An antistatic agent for insulating polymer materials, **characterized in that** the antistatic agent is composed of a donor-acceptor type molecular compound represented by the general formula (1) below and obtained by mixing, melting, and reacting a semi-polar organoboron compound (donor component) of the upper part in the general formula (1) below with a basic nitrogen compound (acceptor component) of the lower part in the general formula (1) below, 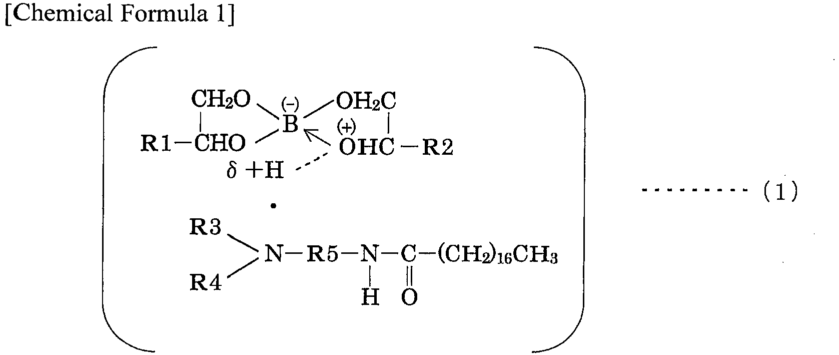 wherein R1 and R2 are each independently CH₃(CH₂)₁₆-CO-OCH₂ or HOCH₂ and at least one thereof is CH₃(CH₂)₁₆-CO-OCH₂; R3 and R4 are each independently CH₃, C₂H₅, HOCH₂, HOC₂H₄, or HOCH₂CH(CH₃); and R5 is C₂H₄ or C₃H₆.

2. A method of producing a molded article comprising an insulating polymer material, **characterized in that** the method comprises: melt-dispersing the antistatic agent according to claim 1 in an insulating polymer material that has been heated to equal to or higher than its glass transition temperature; and carrying out molding in a molding apparatus having a steel surface wherein the insulating polymer material is brought into contact with the steel surface of the molded apparatus and at the surface of the molded article, an antistatic film in which the antistatic agent is arrayed is formed.

3. A molded article comprising an insulating polymer material, **characterized in that** the molded article is produced by the production method according to claim 2 and has, at its surface, the antistatic film in which the antistatic agent composed of the donor-acceptor type molecular compound represented by the general formula (1) is arrayed, 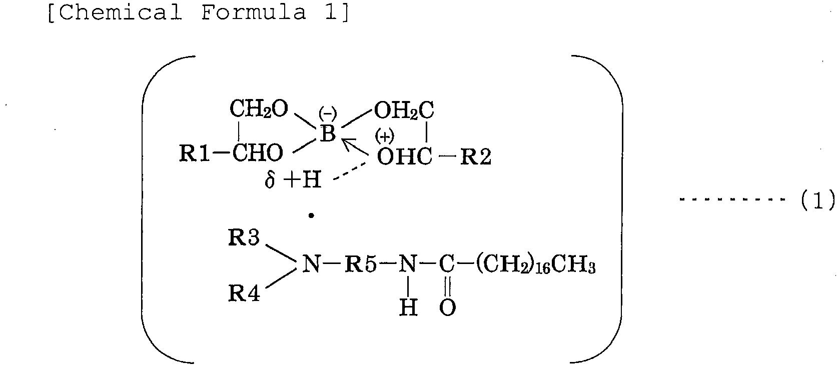 wherein R1 and R2 are each independently CH₃(CH₂)₁₆-CO-OCH₂ or HOCH₂ and at least one thereof is CH₃(CH₂)₁₆-CO-OCH₂; R3 and R4 are each independently CH₃, C₂H₅, HOCH₂, HOC₂H₄, or HOCH₂CH (CH₃); and R5 is C₂H₄ or C₃H₆.

## Patentansprüche

1. Antistatisches Mittel zum Isolieren von Polymermaterialien, **dadurch gekennzeichnet, dass** das antistatische Mittel aus einer molekularen Verbindung vom Donor/Akzeptor-Typ, dargestellt durch die allgemeine Formel (1), zusammengesetzt ist und durch Mischen, Schmelzen und Umsetzen einer semipolaren Organoborverbindung (Donor-Komponente) aus dem oberen Teil in der allgemeinen Formel (1) nachstehend mit einer basischen Stickstoffverbindung (Akzeptor-Komponente) aus dem unteren Teil in der allgemeinen Formel (1) nachstehend erhalten wird, 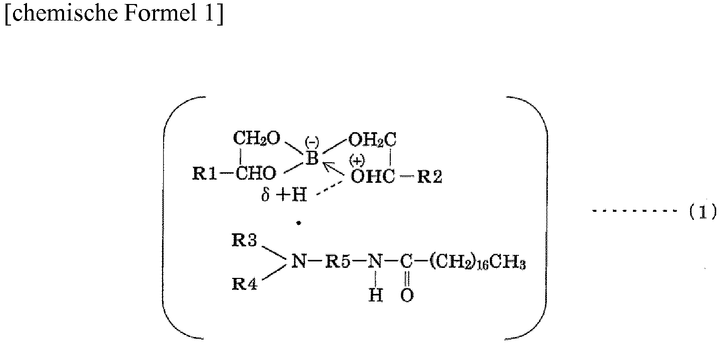 wobei R1 und R2 jeweils unabhängig voneinander CH₃(CH₂)₁₆-CO-OCH₂ oder HOCH₂ sind und mindestens einer davon CH₃(CH₂)₁₆-CO-OCH₂ ist; R3 und R4 jeweils unabhängig voneinander CH₃, C₂H₅, HOCH₂, HOC₂H₄ oder HOCH₂CH(CH₃) sind; und R5 C₂H₄ oder C₃H₆ ist.

2. Verfahren zum Herstellen eines geformten Gegenstandes, umfassend ein isolierendes Polymermaterial, **dadurch gekennzeichnet, dass** das Verfahren umfasst: Schmelzdispergieren des antistatischen Mittels gemäß Anspruch 1 in einem isolierenden Polymermaterial, welches auf seine Glasübergangstemperatur oder höher erwärmt wurde; und Durchführen von Formgebung in einem Formgebungsgerät mit einer Stahloberfläche, wobei das isolierende Polymermaterial mit der Stahloberfläche des Formgebungsgeräts in Kontakt gebracht wird und an der Oberfläche des geformten Gegenstandes ein antistatischer Film, in welchem das antistatische Mittel angeordnet ist, gebildet wird.

3. Geformter Gegenstand, umfassend ein isolierendes Polymermaterial, **dadurch gekennzeichnet, dass** der geformte Gegenstand durch das Herstellungsverfahren gemäß Anspruch 2 hergestellt wird und an seiner Oberfläche den antistatischen Film aufweist, in welchem das antistatische Mittel, das aus der molekularen Verbindung vom Donor/Akzeptor-Typ, dargestellt durch die allgemeine Formel (1), zusammengesetzt ist, angeordnet ist, 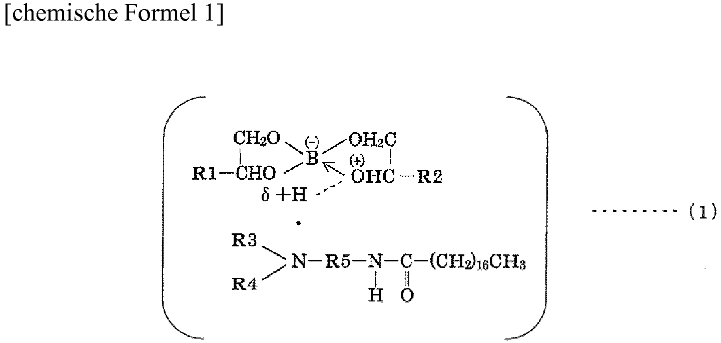 wobei R1 und R2 jeweils unabhängig voneinander CH₃(CH₂)₁₆-CO-OCH₂ oder HOCH₂ sind und mindestens einer davon CH₃(CH₂)₁₆-CO-OCH₂ ist; R3 und R4 jeweils unabhängig voneinander CH₃, C₂H₅, HOCH₂, HOC₂H₄ oder HOCH₂CH(CH₃) sind; und R5 C₂H₄ oder C₃H₆ ist.

## Revendications

1. Agent antistatique pour matériaux polymères isolants, **caractérisé en ce que** l'agent antistatique est composé d'un composé moléculaire de type donneur-accepteur représenté par la formule générale (1) ci-dessous et obtenu par mélange, fusion et réaction d'un composé organoboré semi-polaire (composant donneur) de la partie supérieure de la formule générale (1) ci-dessous avec un composé azoté basique (composant accepteur) de la partie inférieure de la formule générale (1) ci-dessous, 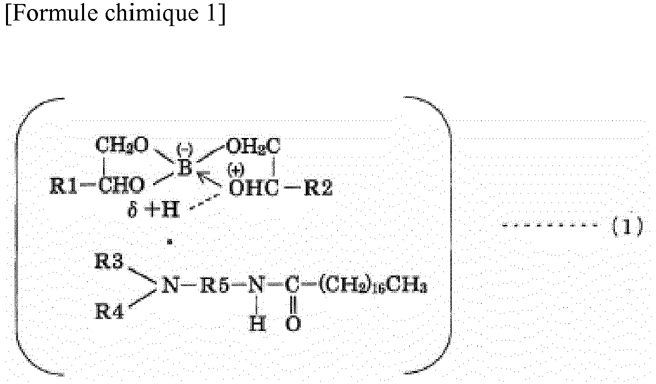 dans laquelle R1 et R2 représentent chacun indépendamment CH₃(CH₂)₁₆-CO-OCH₂ ou HOCH₂ et au moins l'un de ceux-ci est CH₃(CH₂)₁₆-CO-OCH₂ ; R3 et R4 représentent chacun indépendamment CH₃, C₂H₅, HOCH₂, HOC₂H₄ ou HOCH₂CH(CH₃) ; et R5 représente C₂H₄ ou C₃H₆.

2. Procédé pour produire un article moulé comprenant un matériau polymère isolant,
**caractérisé en ce que** le procédé comprend : la dispersion à l'état fondu de l'agent antistatique selon la revendication 1 dans un matériau polymère isolant qui a été chauffé à une température supérieure ou égale à sa température de transition vitreuse ; et la réalisation d'un moulage dans un appareil de moulage ayant une surface d'acier dans lequel le matériau polymère isolant est amené en contact avec la surface d'acier de l'appareil de moulage et à la surface de l'article moulé, un film antistatique dans lequel est disposé l'agent antistatique est formé.

3. Article moulé comprenant un matériau polymère isolant, **caractérisé en ce que** l'article moulé est produit par le procédé de production selon la revendication 2 et présente, à sa surface, le film antistatique dans lequel l'agent antistatique composé du composé moléculaire de type donneur-accepteur représenté par la formule générale (1) est disposé, 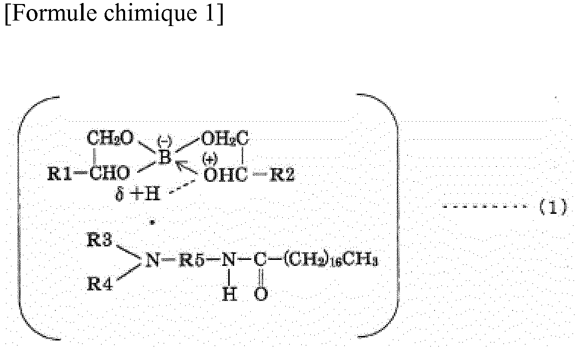 dans laquelle R1 et R2 représentent chacun indépendamment CH₃(CH₂)₁₆-CO-OCH₂ ou HOCH₂ et au moins l'un de ceux-ci est CH₃(CH₂)₁₆-CO-OCH₂ ; R3 et R4 représentent chacun indépendamment CH₃, C₂H₅, HOCH₂, HOC₂H₄ ou HOCH₂CH(CH₃) ; et R5 représente C₂H₄ ou C₃H₆.
